# FASCICULE DE BREVET EUROPEEN

(11) **EP 1 413 287 B1**
(45) Date de publication et mention de la délivrance du brevet: **14.12.2005**
(21) Numéro de dépôt: 03292604.0
(22) Date de dépôt: 20.10.2003
(51) Int. Cl.: A61K 7/13

(54) **Composition de teinture d'oxydation pour fibres kératiniques comprenant un poly (vinyllactame) cationique et au moins un acide gras en C10-C14, procédés et dispositifs de teinture d'oxydation**
Mittel zum oxidativen färben von keratinfasern, enthaltend ein kationisches polyvinyllactam und mindenstens eine ein C10-C14 fettsäure, sowie verfahren und vorrichtungen zur färbung
Oxidation dyeing composition for keratin fibres comprising a cationic poly(vinyllactam)and at least a C10-C14 fatty acid, and the dyeing process and used devices

(30) Priorité: 21.10.2002 FR 0213099
(43) Date de publication de la demande: 28.04.2004
(73) Titulaire: L'OREAL, 75008 Paris (FR)
(72) Inventeur: Cottard, François, 92400 Courbevoie (FR); Rondeau, Christine, 78500 Sartrouville (FR)
(74) Mandataire: Dossmann, Gérard

(56) Documents cités:
- EP-A- 1 179 336
- FR-A- 2 820 032
- US-A- 5 143 518

## Description

La présente invention concerne une composition de teinture d'oxydation des fibres kératiniques, en particulier des fibres kératiniques humaines et plus particulièrement des cheveux, comprenant au moins un colorant d'oxydation, au moins un poly(vinyllactame) cationique et au moins un acide gras particulier.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Pour localiser le produit de coloration d'oxydation à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, certains polyuréthanes, les cires ou encore à des mélanges d'agents tensio-actifs non-ioniques de HLB (Hydrophilic Lipophilic Balance), convenablement choisie qui engendrent l'effet gélifiant quand on les dilue au moyen d'eau et/ou d'agents tensio-actifs.

La plupart des systèmes épaississants de l'art antérieur ne permet pas d'obtenir des nuances puissantes et chromatiques de faibles sélectivités et de bonnes ténacités, et d'assurer un bon état cosmétique à la chevelure traitée. Par ailleurs, il a été constaté que la plupart des compositions tinctoriales prêtes à l'emploi de l'art antérieur contenant au moins un colorant d'oxydation, et un système épaississant ne permettent pas une application suffisamment précise sans coulures ni chutes de viscosité dans le temps.

La demande de brevet FR 2 820 032 décrit des compositions de teinture d'oxydation prêtes à l'emploi qui ne coulent pas et restent donc bien localisées au point d'application, ces compositions comprennent, dans un milieu approprié pour la teinture, au moins un colorant d'oxydation, et au moins un poly(vinyllactame) cationique, elles permettent aussi d'obtenir des nuances puissantes et chromatiques (lumineuses) avec de faibles sélectivités et de bonnes ténacités vis-à-vis des agents chimiques (shampooing, permanentes ...) ou naturels (lumière, transpiration ...) tout en apportant aux cheveux de bonnes propriétés cosmétiques.

Les compositions comprenant au moins un colorant d'oxydation et un système épaississant peuvent se présenter sous la forme de crèmes. La technologie actuelle en matière de colorant d'oxydation implique alors que ces compositions, pour acquérir un aspect crème, comprennent des teneurs élevées d'actifs gras (alcools, amides, acides).

Cependant, la demanderesse a constaté que la viscosité de ces crèmes évoluant lors de leur conservation, il est difficile d'obtenir un mélange homogène lorsque l'on mélange ces compositions sous forme de crème avec un agent oxydant. En outre, la consistance de ces crèmes les rend difficiles à utiliser.

De manière avantageuse et surprenante, la demanderesse a découvert qu'il était possible d'obtenir des compositions de teinture d'oxydation prêtes à l'emploi qui présentent une facilité de mélange accrue avec l'agent oxydant et les autres composants éventuels, une amélioration des propriétés moussantes et une facilité d'élimination accrue, en particulier au rinçage.

En outre, ces compositions selon l'invention ne coulent pas et restent donc bien localisées au point d'application, elles permettent aussi d'obtenir des nuances puissantes et chromatiques (lumineuses) avec des faibles sélectivités et de bonnes ténacités vis-à-vis des agents chimiques (shampooings, permanentes ...) ou naturels (lumière, transpiration ...) tout en apportant aux cheveux de bonnes propriétés cosmétiques.

Il a également été constaté que les compositions selon l'invention peuvent présenter des teneurs en actifs gras réduites par rapport aux teneurs des compositions de l'art antérieur sans que la consistance de la composition (crème) en soit affectée.

La présente invention a ainsi pour objet une composition de teinture d'oxydation pour fibres kératiniques, en particulier pour fibres kératiniques humaines, et en particulier les cheveux, comprenant, dans un milieu approprié à la teinture, au moins un colorant d'oxydation, au moins un acide gras en C₁₀-C₁₄ et au moins un poly(vinyllactame) cationique.

Un autre objet de l'invention porte sur une composition prête à l'emploi pour la teinture des fibres kératiniques qui contient au moins un colorant d'oxydation, au moins un acide gras en C₁₀-C₁₄, et au moins un poly(vinyllactame) cationique tel que défini ci-après et un agent oxydant.

Par "composition prête à l'emploi", on entend, au sens de l'invention, la composition destinée à être appliquée telle quelle sur les fibres kératiniques, c'est-à-dire qu'elle peut être stockée telle quelle avant utilisation ou résulter du mélange extemporané de deux ou plusieurs compositions.

Une quantité efficace de poly(vinyllactame) cationique est ainsi introduite :
(i) soit dans la composition contenant le ou les colorants d'oxydation et éventuellement le ou les coupleurs (ou composition A),
(ii) soit dans la composition oxydante (ou composition B), ou
(iii) dans les deux compositions à la fois.

Une quantité efficace d'acide gras en C₁₀-C₁₄ est introduite
(i) soit dans la composition contenant le ou les colorants d'oxydation et éventuellement le ou les coupleurs (ou composition A),
(ii) soit dans la composition oxydante (ou composition B), ou
(iii) dans les deux compositions à la fois.

De préférence, l'acide gras en C₁₀-C₁₄ est dans la composition contenant le ou les colorants d'oxydation.

L'invention vise également un procédé de teinture des fibres kératiniques, et en particulier des fibres kératiniques humaines telles que les cheveux, consistant à appliquer sur les fibres au moins une composition A contenant, dans un milieu approprié pour la teinture, au moins un colorant d'oxydation, la couleur étant révélée à pH alcalin, neutre ou acide, à l'aide d'une composition B contenant au moins un agent oxydant qui est mélangée juste au moment de l'emploi à la composition A ou qui est appliquée séquentiellement sans rinçage intermédiaire, au moins un poly(vinyllactame) cationique tel que défini ci-après étant présent dans la composition A ou dans la composition B ou dans chacune des compositions A et B et au moins un acide en C₁₀-C₁₄ tel que défini ci-après étant présent dans la composition A ou dans la composition B ou dans chacune des compositions A et B.

L'invention a également pour objet des dispositifs de teinture ou « kits » à plusieurs compartiments.

Un dispositif à 2 compartiments selon l'invention comprend un compartiment renfermant une composition A1 contenant, dans un milieu approprié pour la teinture, au moins un colorant d'oxydation, et un autre compartiment renfermant une composition B1 contenant, dans un milieu approprié pour la teinture, un agent oxydant, le polymère poly(vinyllactame) cationique tel que défini ci-après étant présent dans la composition A1 ou la composition B1, ou dans chacune des compositions A1 et B1 et l'acide en C₁₀-C₁₄ tel que défini ci-après étant présent dans la composition A1 ou la composition B1, ou dans chacune des compositions A1 et B1.

Un autre dispositif, à 3 compartiments selon l'invention, comprend un premier compartiment renfermant une composition A2 contenant, dans un milieu approprié pour la teinture, au moins un colorant d'oxydation, un deuxième compartiment renfermant une composition B2 contenant, dans un milieu approprié pour la teinture au moins un agent oxydant, et un troisième compartiment renfermant une composition C contenant, dans un milieu approprié pour la teinture, au moins un polymère poly(vinyllactame) cationique, la composition A2 et/ou la composition B2 pouvant également contenir un polymère poly(vinyllactame) cationique tel que défini ci-après et la composition A2 et/ou la composition B2 et/ou la composition C pouvant également contenir un acide gras en C₁₀-C₁₄ tel que défini ci-après.

D'autres caractéristiques, aspects, objets et avantages de l'invention apparaîtront encore plus clairement à la lecture de la description et des exemples qui suivent.

Les polymères associatifs sont des polymères dont les molécules sont capables, dans le milieu de formulation, de s'associer entre elles ou avec des molécules d'autres composés.

Leur structure chimique comprend généralement au moins une zone hydrophile et au moins une zone hydrophobe, la ou les zones hydrophobes comprenant au moins une chaîne grasse.

### Polymères polyvinyllactames cationiques selon l'invention

Les polymères poly(vinyllactame) cationiques utilisés selon l'invention comprennent :
-a) au moins un monomère de type vinyl lactame ou alkylvinyllactame;
-b) au moins un monomère de structures (Ia) ou (Ib) suivantes : 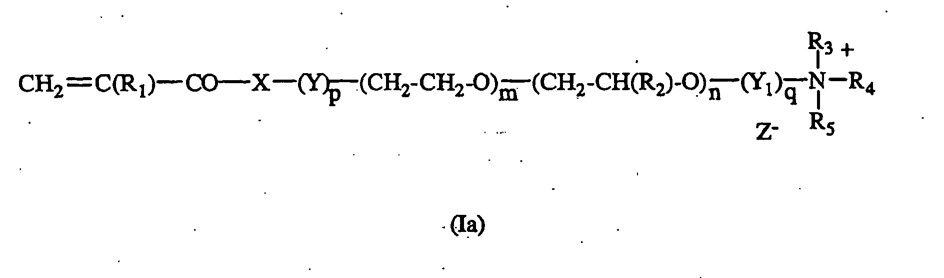 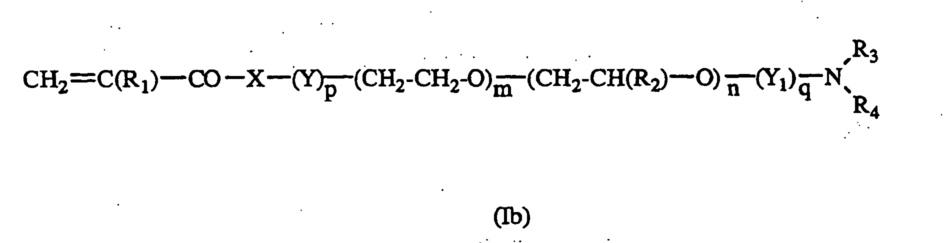
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (II) : 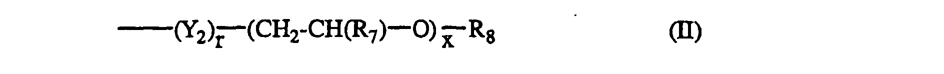
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Les polymères poly(vinyllactame) cationiques utilisés selon l'invention peuvent être réticulés ou non réticulés et peuvent aussi être des polymères blocs.

De préférence, le contre ion Z⁻ des monomères de formule (Ia) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (Ia) pour laquelle, encore plus préférentiellement, m et n sont égaux à zéro.

Le monomère vinyl lactame ou alkylvinyllactame est de préférence un composé de structure (III) : 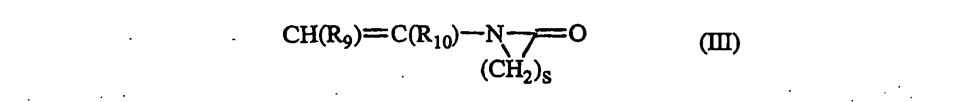 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (III) est la vinylpyrrolidone.

Les polymères poly(vinyllactame) cationiques utilisés selon l'invention peuvent également contenir un ou plusieurs monomères supplémentaires, de préférence cationiques ou non ioniques.

A titre de composés utilisés de manière plus particulièrement préférée selon l'invention, on peut citer les terpolymères suivants comprenant au moins :
a)-un monomère de formule (III),
b)-un monomère de formule (Ia) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (Ib) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b).

De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone /diméthylaminopropylméthàcrylamide / tosylate de dodécyldiméthylméthacrylamidopropylammonium, les terpolymères vinylpyrrolidone /diméthylaminopropylméthacrylamide/ tosylate de cocoyldiméthylméthacrylamidopropylammonium, les terpolymères vinylpyrrolidone /diméthylaminopropylméthacrylamide / tosylate ou chlorure de lauryldiméthylméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Dans la ou les composition(s) de teinture selon l'invention, le ou les poly(vinyllactame) cationiques décrits ci-dessus sont utilisés de préférence en une quantité pouvant varier d'environ 0,01 à 10% en poids du poids total de la composition. Plus préférentiellement, cette quantité varie d'environ 0,1 à 5% en poids.

De préférence la viscosité des compositions selon l'invention est supérieure à 1000 cp, mesurée à 25°C à l'aide d'un rhéomètre RHEOMAT RM 180 à un taux de cisaillement de 200 s⁻¹.

### Acides gras en C₁₀-C₁₄

Les acides gras utilisés dans la composition selon la présente invention sont des acides gras en C₁₀-C₁₄.

Il pourra, par exemple, s'agir des acides caprique, laurique, myristique, et leurs mélanges.

De manière avantageuse, on utilisera l'acide laurique.

Ces acides gras en C₁₀-C₁₄ peuvent être libres, partiellement ou totalement neutralisés dans les compositions de l'invention. De préférence, ils sont totalement neutralisés.

Dans la composition de teinture selon l'invention, le ou les acides gras en C₁₀-C₁₄ sont utilisés de préférence en quantité pouvant varier d'environ 0,1 % à 40% en poids du poids total de la composition. Plus préférentiellement, cette quantité varie d'environ 2 % à 25%, et encore plus préférentiellement de 5% à 20% en poids.

### Colorants d'oxydation

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les paraphénylènediamines, les bases doubles, les ortho- et para-aminophénols, les bases hétérocycliques suivantes ainsi que leurs sels d'addition avec un acide .

On peut notamment citer :
- (A) les paraphénylènediamines de formule (IV) suivante et leurs sels d'addition avec un acide : 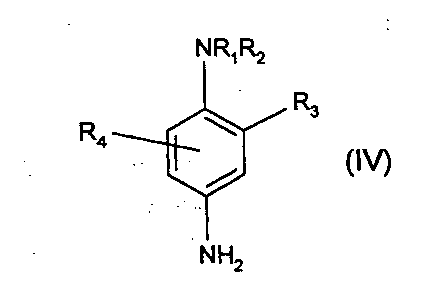 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

   Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (IV) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl) amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylamino éthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (IV) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (B) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (V) suivante, et leurs sels d'addition avec un acide : 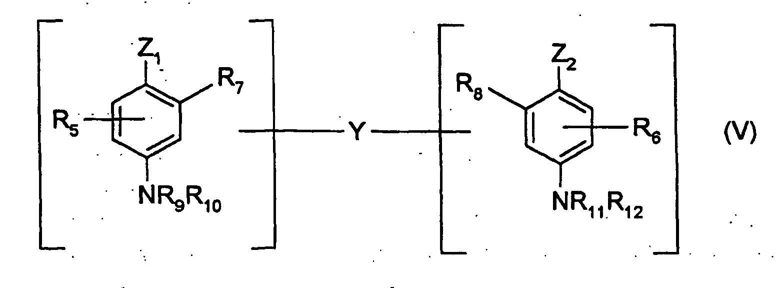 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (V) ne comportent qu'un seul bras de liaison Y par molécule.
   Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (V) ci-dessus, on peut plus particulièrement citer le N,N'-bis-(β-hydroxyéthyl)-N,N'-bis-(4'-aminophényl)-1,3-diamino-propanol, la N,N-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (V), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (C) les para-aminophénols répondant à la formule (VI) suivante, et leurs sels d'addition avec un acide : 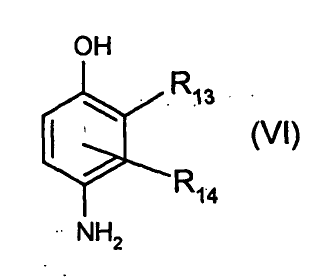 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (VI) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (D) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (E) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la. 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 20% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, lés pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent chacun de préférence de 0,0001 à 20% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition A et/ou la composition B et/ou la composition C peuvent en outre plus particulièrement contenir, au moins un polymère amphotère ou un polymère cationique différent des poly(vinyllactame) cationiques selon la présente invention.

### Polymères cationiques différents de ceux de l'invention

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (VII), (VIII), (IX) ou (X) suivantes: 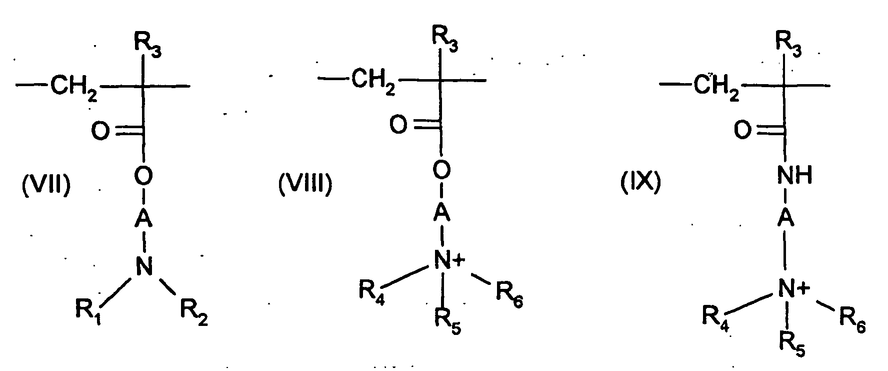 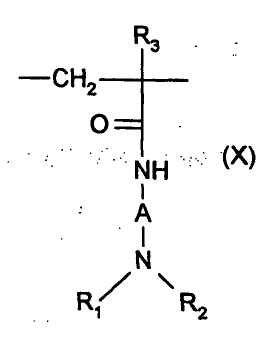 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   les espèces chargées de formule (VIII) et (IX) sont associées à un contre-ion X⁻,
   X⁻ désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC® par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100® par la société CIBA GEIGY,
   - le copolymère d'acrylamide et de méthosulfate de méthacryloyloxyéthyltriméthylammonium vendu sous la dénomination RETEN® par la société HERCULES,
   - les copolymères vinylpyrrolidone / acrylate ou méthacrylate de dialkylaminoalkyle quaternisés ou non, tels que les produits vendus sous la dénomination "GAFQUAT®" par la société ISP comme par exemple "GAFQUAT 734" ou "GAFQUAT 755" ou bien les produits dénommés "COPOLYMER 845®, 958® et 937®". Ces polymères sont décrits en détail dans les brevets français 2.077.143 et 2.393.573,
   - les terpolymères méthacrylate de diméthyl amino éthyle/vinylcaprolactame/ vinylpyrrolidone tel que le produit vendu sous la dénomination GAFFIX VC 713® par la société ISP,
   - les copolymère vinylpyrrolidone / méthacrylamidopropyl dimethylamine commercialisés notamment sous la dénomination STYLEZE CC 10® par ISP,
   - et les copolymères vinylpyrrolidone / méthacrylamide de diméthylaminopropyle quaternisés tel que le produit vendu sous la dénomination "GAFQUAT® HS 100" par la société ISP .
(2) Les dérivés d'éthers de cellulose comportant des groupements ammonium quaternaire décrits dans le brevet français 1 492 597, et en particulier les polymères commercialisés sous les dénominations "JR®" (JR 400, JR 125, JR 30M) ou "LR®" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat® L 200" et "Celquat® H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR® C13 S, JAGUAR® C 15, JAGUAR® C 17 ou JAGUAR® C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine® F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone: Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett® 57" par la société Hercules Inc. ou bien sous la dénomination de "PD 170®" ou "Delsette 101®" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (XI) ou (XII) : 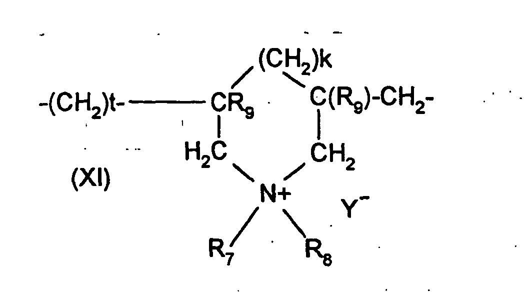 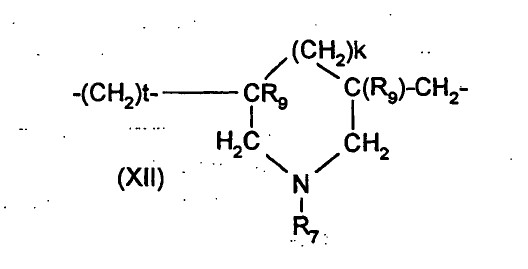 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat® 100" par la société Calgon (et ses homologues de faible masse moléculaire moyenne en poids) et les copolymères de chlorure de diallyldiméthylammonium et d'acrylamide commercialisés sous la dénomination "MERQUAT® 550".
(10) Le polymère de diammonium quaternaire contenant des motifs récurrents répondant à la formule : 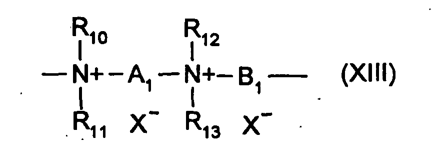 formule (XIII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (XIV) suivante : 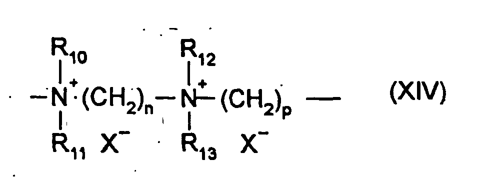 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(11) Les polymères de polyammonium quaternaire constitués de motifs récurrents de formule (XV) : 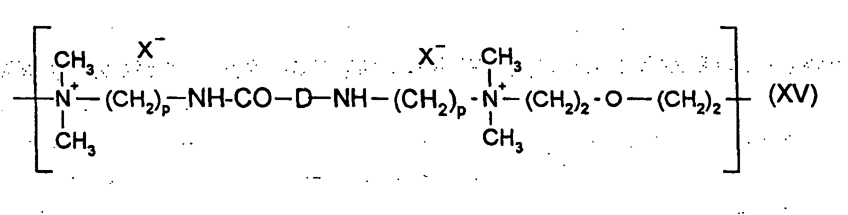 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol® A 15", "Mirapol® AD1", "Mirapol® AZ1" et "Mirapol® 175" vendus par la société Miranol.
(12) Les polymères quaternaires de vinylpyrrolidone et de vinylimidazole tels que par exemple les produits commercialisés sous les dénominations Luviquat® FC 905, FC 550 et FC 370 par la société B.A.S.F.
(13) Les polyamines comme le Polyquart H® vendu par HENKEL, référencé sous le nom de " POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 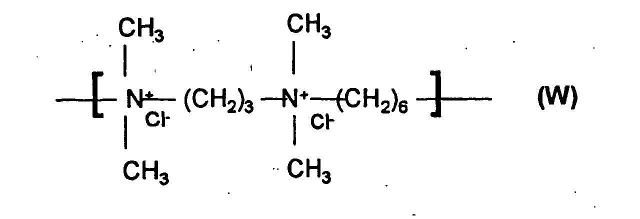 et notamment ceux dont le poids moléculaire, déterminé par chromatographie par perméation de gel, est compris entre 9500 et 9900; 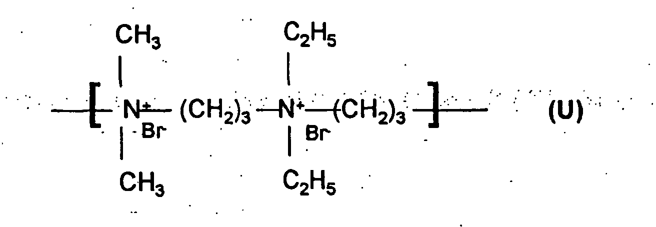 et notamment ceux dont le poids moléculaire, déterminé par chromatographie par perméation de gel, est d'environ 1200.

Le ou les polymères cationiques additionnels sont présents dans la composition selon la présente invention dans une concentration pouvant varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART® KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT® 280, MERQUAT® 295 et MERQUAT® PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER® ou LOVOCRYL® 47 par la société NATIONAL STARCH.
(3) Les polyaminoamides réticulés et alcoylés partiellement ou totalement dérivant de polyaminoamides de formule générale :

   ⁅CO-R₁₉-CO-Z-⁆ (XVI)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 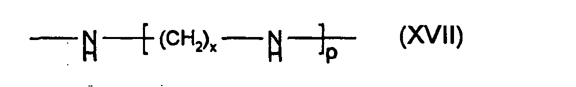 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XVII) ci-dessus, dans lequel x=2 et p=1 et qui dérive de l'éthylènediamine, ou le radical dérivant de la pipérazine : 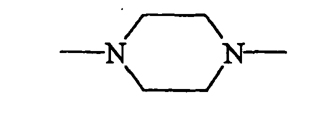
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 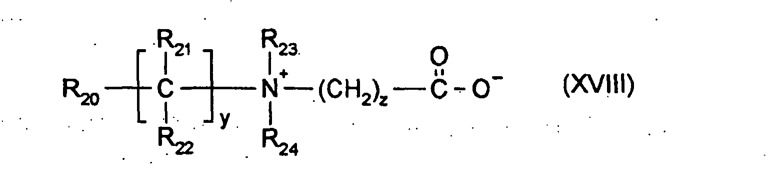 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301® par la société SANDOZ.
(5) les polymères dérivés du chitosane comportant des motifs monomères répondant aux formules (XIX), (XX), (XXI) suivantes : 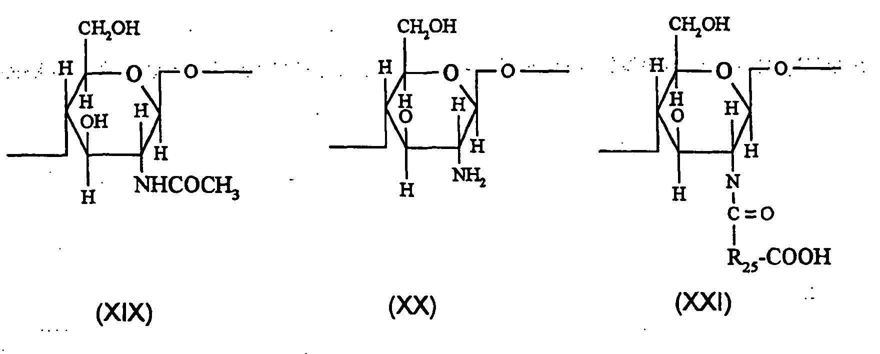 le motif (XIX) étant présent dans des proportions comprises entre 0 et 30%, le motif (XX) dans des proportions comprises entre 5 et 50% et le motif (XXI) dans des proportions comprises entre 30 et 90%, étant entendu que dans ce motif (XXI), R₂₅ représente un radical de formule : 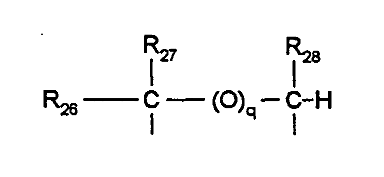 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN®" par la société JAN DEKKER.
(7) Les polymères répondant à la formule générale (XXII) tels que ceux décrits par exemple dans le brevet français 1 400 366 : 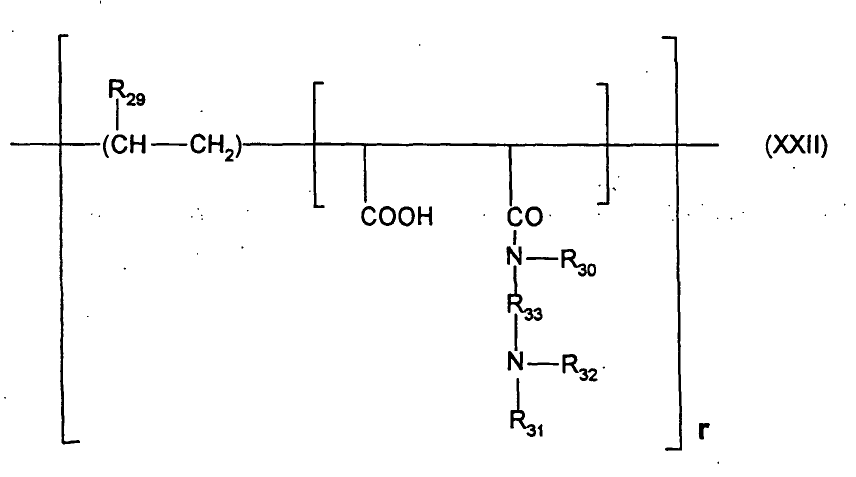 dans laquelle R₂₉ représente un atome d'hydrogène, un radical CH₃O, CH₃CH₂O, phényle, R₃₀ désigne l'hydrogène ou un radical alkyle inférieur tel que méthyle, éthylé, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XXIII)

      où D désigne un radical 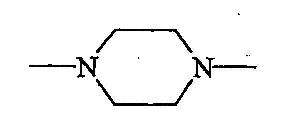 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XXIV)

      où D désigne un radical 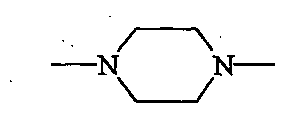
   et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères additionnels peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs autres que les acides gras en C₁₀-C₁₄.

Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates; ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative), les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le -cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

### Agents épaississants additionnels

Les compositions selon l'invention peuvent également contenir d'autres agents d'ajustement de la rhéologie tels que les épaississants cellulosiques (hydroxyéthylcellulose, hydroxypropylcellulose, carboxyméthylcellulose...), la gomme de guar et ses dérivés (hydroxypropylguar...), les gommes d'origine microbienne (gomme de xanthane, gomme de scléroglucane...), les épaississants synthétiques tels que les homopolymères réticulés d'acide acrylique ou d'acide acrylamidopropanesulfonique et les polymères associatifs ioniques ou non ioniques tels que les polymères commercialisés sous les appellations PEMULEN® TR1 ou TR2 par la société GOODRICH, SALCARE® SC90 par la société ALLIED COLLOIDS, ACULYN® 22, 28, 33, 44, ou 46 par la société ROHM & HAAS et ELFACOS® T210 et T212 par la société AKZO.

Ces épaississants d'appoint peuvent représenter de 0,01 à 10% en poids du poids total de la composition.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations allant d'environ 0,5 à 20% et, de préférence, d'environ 2 à 10% en poids par rapport au poids total de la composition.

La composition A peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des polymères associatifs autres que ceux de la présente invention, et en particulier des polyuréthanes polyéthers associatifs non-ioniques.

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition B, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale A et de la composition oxydante B et éventuellement de la composition C], est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XXV) suivante : 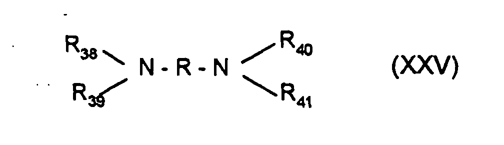 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions A et B et éventuellement C décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Selon lesdits procédés, les compositions A, et/ou B peuvent contenir en outre au moins un polymère cationique ou amphotère additionnel et au moins un tensio-actif.

Un exemple concret illustrant l'invention est indiqué ci-après, sans pour autant présenter un caractère limitatif.

### EXEMPLE :

On a préparé les compositions suivantes :
(quantités exprimées en grammes)

### Composition oxydante :

| | |
|---|---|
| Mélange alcool cétylstéarylique (80%) / alcool cétylstéarylique à 30 OE (20%) (SINNOWAX AO de COGNIS) | 2,35 g |
| Diéthanolamide d'acide oléique | 0,95 g |
| Glycérine | 0,5 g |
| Péroxyde d'hydrogène à 50% en solution dans l'eau | 12 g |
| Séquestrant | 0,15 g |
| Agents stabilisants | 0,125 g |
| Parfum | qs |
| Agents acidifiants | Qs pH 2,8 |
| Eau déminéralisée qsp | 100 g |

### Composition colorante:

| | |
|---|---|
| Acide laurique naturel | 2,5 |
| Alcool laurique oxyéthyléné (12 OE) | 7,5 |
| Alcool cétylstéarylique (C16/C18 50/50) | 10 |
| Monostéarate de glycol | 2 |
| Alcool oléocétylique oxyéthyléné (30 OE) | 3 |
| Alcool décylique oxyéthyléné (3 OE) | 10 |
| Silice pyrogénée à caractère hydrophobe | 1 |
| Monoéthanolamine pure | 1,2 |
| Homopolymère chlorure de diméthyl diallyl ammonium en solution aqueuse à 40% | 7 |
| Propylène glycol | 10 |
| Terpolymère de vinylpyrrolidone, diméthylaminopropylméthacrylamide et de chlorure de lauryldiméthyl-propylméthacrylamido ammonium (74/15/11) | 4 |
| Acide polyacrylique réticulé | 0,4 |
| Acide diéthylène triamine pentacétique, sel pentasodique en solution aqueuse à 40% | 2 |
| Thiolactate d'ammonium en solution aqueuse à 58% (50% en acide thiolactique) | 0,8 |
| Mono-tertiobutyl hydroquinone | 0,3 |
| 1,4-diamino-benzène | 0,24 |
| 1-hydroxy-4-amino-benzène | 0,44 |
| 1-hydroxy-2-amino-benzène | 0,028 |
| 1,3-dihydroxybenzène (Resorcinol) | 0,192 |
| 1-hydroxy-3-amino-benzène | 0,019 |
| 1-méthyl-2-hydroxy-4-béta-hydroxyéthylamino-benzène | 0,021 |
| 2-méthyl-1,3-dihydroxybenzène (2-méthylrésorcinol) | 0,055 |
| Ammoniaque (à 20,5% en ammoniac) | 10 |
| Parfum | 0,5 |
| Eau désionisée (QS) | Qsp 100 |

Le polymère utilisé selon l'invention est un terpolymère vinylpyrrolidone / diméthylaminopropylméthacrylamide/ chlorure de lauryldiméthylméthacrylamidoammonium proposé par la Société ISP sous la référence POLYMER ACP-1234.

La composition colorante a été mélangée, au moment de l'emploi, dans un bol en plastique et pendant 2 minutes, à la composition oxydante donnée ci-dessus, à raison de 1 partie de composition colorante pour 1,5 parties de composition oxydante.

Le mélange obtenu est onctueux et se prépare aisément.

Il est appliqué sur des cheveux naturels à 90% de blancs. On laisse poser 30 minutes.

Le produit s'élimine aisément par rinçage à l'eau

Après lavage avec un shampooing standard, les cheveux sont séchés. Ils sont alors teints dans une nuance blond doré.

Si l'on substitue l'acide laurique naturel par une quantité équivalente d'acide stéarylique, on obtient un mélange moins aisé à préparer, moins agréable à appliquer et qui s'élimine plus difficilement. La nuance obtenue est de qualité inférieure.

## Revendications

1. Composition de teinture d'oxydation pour fibres kératiniques, en particulier pour fibres kératiniques humaines et plus particulièrement des cheveux, comprenant dans un milieu approprié pour la teinture, au moins un colorant d'oxydation, au moins un acide gras en C₁₀-C₁₄ et au moins un polymère poly(vinyllactame) cationique comprenant :
-a) au moins un monomère de type vinyl lactame ou alkylvinyllactame;
-b) au moins un monomère de structures (Ia) ou (Ib) suivantes : 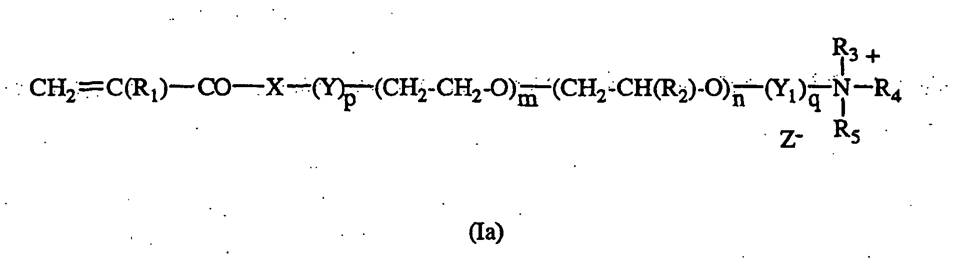 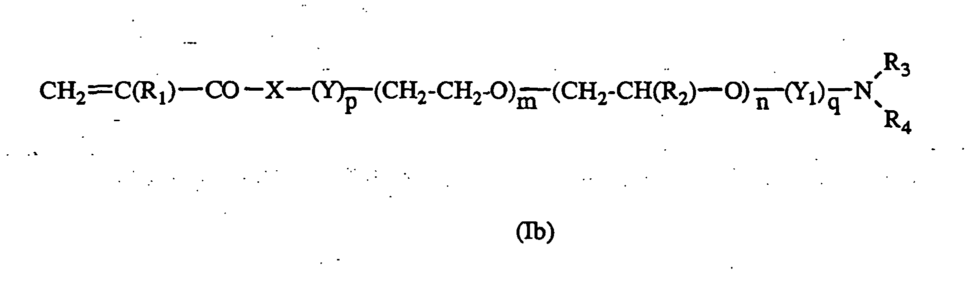
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄;
R₃, R₄ et R₅ désignent, indépendamment l'un de l'autre, un atome d'hydrogène, un radical alkyle linéaire ou ramifié en C₁-C₃₀ ou un radical de formule (II): 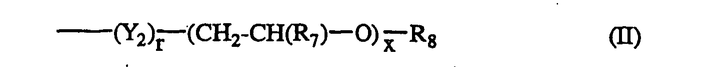
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

2. Composition selon la revendication 1 **caractérisée par le fait que** le monomère vinyl lactame ou alkylvinyllactame est un composé de structure (III) : 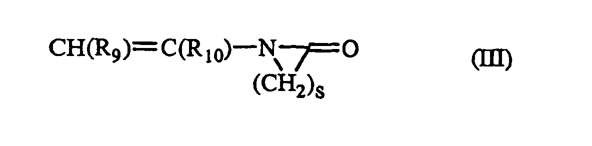 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

3. Composition selon la revendication 2 **caractérisée par le fait que** le monomère de formule (III) est la vinylpyrrolidone.

4. Composition selon l'une quelconque des revendications précédentes, **caractérisée par le fait que** dans les formules (Ia) ou (Ib), les radicaux R₃, R₄, R₅ désignent, indépendamment l'un de l'autre, un atome d'hydrogène ou un radical alkyle linéaire ou ramifié en C₁-C₃₀.

5. Composition selon l'une quelconque des revendications précédentes, **caractérisée par le fait que** le monomère b) est un monomère de formule (Ia).

6. Composition selon la revendication 5, **caractérisée par le fait que** dans la formule (Ia), m et n sont égaux à zéro.

7. Composition selon l'une quelconque des revendications précédentes, **caractérisée par le fait que** le contre ion Z-des monomères de formule (Ia) est choisi parmi les ions halogénures, les ions phosphates, l'ion méthosulfate, l'ion tosylate.

8. Composition selon l'une quelconque des revendications 1 à 7, **caractérisée par le fait que** le ou les polymères poly(vinyllactame) cationiques contiennent un ou plusieurs monomères supplémentaires cationiques ou non ioniques.

9. Composition selon la revendication 8, **caractérisée par le fait que** le poly(vinyllactame) cationique est un terpolymère comprenant :
a)-un monomère de formule (III),
b)-un monomère de formule (Ia) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (Ib) dans laquelle R₃ et R₄ désignent, indépendamment l'un de l'autre, un atome d'hydrogène ou un radical alkyle en C₁-C₅.

10. Composition selon la revendication 9, **caractérisée par le fait que** le terpolymère comprend en poids, 40 à 95% de monomère (a), 0,25 à 50% de monomère (b) et 0,1 à 55% de monomère (c).

11. Composition selon l'une quelconque des revendications précédentes, **caractérisée par le fait que** les poly(vinyllactame) cationiques sont choisis parmi les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.

12. Composition selon l'une quelconque des revendications précédentes, **caractérisée par le fait que** la masse moléculaire en poids des poly(vinyllactame) cationiques est comprise entre 500 et 20 000 000, de préférence comprise entre 200 000 et 2 000 000 et plus préférentiellement comprise entre 400 000 et 800 000.

13. Composition selon l'une quelconque des revendications précédentes, **caractérisée par** le fait le ou les poly(vinyllactame) cationiques sont utilisés en une quantité variant de 0,01 à 10% en poids du poids total de la composition.

14. Composition selon la revendication 13, **caractérisée par** le fait le ou les poly(vinyllactame) cationiques sont utilisés en une quantité variant de 0,1 à 5% en poids du poids total de la composition.

15. Composition selon l'une quelconque des revendications 1 à 14, **caractérisée par le fait que** l'acide gras en C₁₀-C₁₄ est choisi dans le groupe formé par les acides caprique, laurique et myristique et leurs mélanges.

16. Composition selon la revendication 15, **caractérisé en ce que** l'acide gras en C₁₀-C₁₄ est l'acide laurique.

17. Composition selon l'une quelconque des revendications précédentes, **caractérisée par le fait que** le ou les acides gras en C₁₀-C₁₄ sont utilisés en une quantité variant de 0,1 à 40%, de préférence de 2 à 25% et encore plus préférentiellement de 5 à 20% en poids par rapport au poids total de la composition.

18. Composition selon l'une quelconque des revendications précédentes, **caractérisée par le fait que** le colorant d'oxydation est choisi parmi les bases d'oxydation et/ou les coupleurs.

19. Composition selon la revendication 18, **caractérisée par le fait qu'**elle contient au moins une base d'oxydation.

20. Composition selon les revendications 18 ou 19, **caractérisée par le fait que** les bases d'oxydation sont choisies parmi les para- phénylènediamines, les bases doubles, les ortho- ou para-aminophénols, et les bases hétérocycliques, ainsi que les sels d'addition de ces composés avec un acide.

21. Composition selon l'une quelconque des revendications 18 à 20, **caractérisée par le fait que** les bases d'oxydation sont présentes chacune dans des concentrations allant de 0,0005 à 20% en poids par rapport au poids total de la composition.

22. Composition selon la revendication 18, **caractérisée par le fait que** les coupleurs sont choisis parmi les métaphénylènediamines, les méta-aminophénols, les métadiphénols, les coupleurs hétérocycliques, et les sels d'addition de ces composés avec un acide.

23. Composition selon l'une quelconque des revendications 18 ou 22, **caractérisée par le fait que** les coupleurs sont présents chacun dans des concentrations allant de 0,0001 à 20% en poids par rapport au poids total de la composition.

24. Composition selon l'une quelconque des revendications 20 ou 22, **caractérisée par le fait que** les sels d'addition avec un acide des colorants d'oxydation sont choisis parmi les chlorhydrates, les bromhydrates, les sulfates, les tartrates, les lactates et les acétates.

25. Composition selon l'une quelconque des revendications précédentes, **caractérisée par le fait qu'**elle contient en outre des colorants directs.

26. Composition selon l'une quelconque des revendications précédentes, **caractérisée par le fait qu'**elle contient en outre au moins un polymère amphotère ou un polymère cationique différent du ou des poly(vinyllactame) cationiques tels que définis à l'une quelconque des revendications 1 à 12.

27. Composition selon la revendication 26, **caractérisée par le fait que** le polymère cationique est un poly(ammonium quaternaire) constitué de motifs récurrents répondant à la formule (W) suivante :

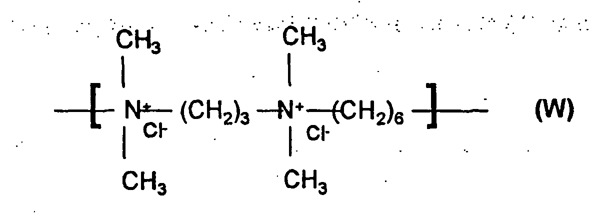

28. Composition selon la revendication 26, **caractérisée par le fait que** le polymère cationique est un poly(ammonium quaternaire) constitué de motifs récurrents répondant à la formule (U) suivante:

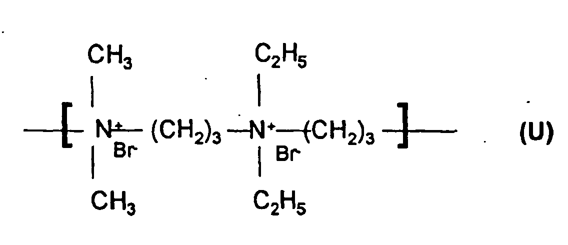

29. Composition selon la revendication 26, **caractérisée par le fait que** le polymère amphotère est un copolymère comprenant au moins comme monomères de l'acide acrylique et un sel de diméthyldiallylammonium.

30. Composition selon l'une quelconque des revendications 26 à 29, **caractérisée par le fait que** le ou les polymères cationiques ou amphotères représentent de 0,01 % à 10 %, de préférence de 0,05 % à 5 %, et encore plus préférentiellement de 0,1 % à 3 % en poids, du poids total de la composition.

31. Composition selon l'une quelconque des revendications précédentes, **caractérisée par le fait qu'**elle contient au moins un tensioactif choisi parmi les tensioactifs anioniques, cationiques, non ioniques ou amphotères.

32. Composition selon la revendication 31, **caractérisée par le fait que** les tensioactifs représentent 0,01 à 40% et de préférence de 0,5 à 30% en poids, du poids total de la composition.

33. Composition selon l'une quelconque des revendications précédentes, **caractérisée par le fait qu'**elle contient au moins un agent épaississant additionnel.

34. Composition selon l'une quelconque des revendications précédentes, **caractérisée par le fait qu'**elle contient en outre au moins un agent réducteur, dans des quantités allant de 0,05 à 3% en poids par rapport au poids total de la composition.

35. Composition selon l'une quelconque des revendications précédentes, **caractérisée par le fait qu'**elle contient en outre un agent oxydant et qu'elle est prête à l'emploi.

36. Composition selon la revendication 35, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

37. Composition selon la revendication 36, **caractérisée par le fait que** l'agent oxydant est le peroxyde d'hydrogène.

38. Composition selon la revendication 37, **caractérisée par le fait que** l'agent oxydant est une solution d'eau oxygénée dont le titre varie de 1 à 40 volumes.

39. Composition selon la revendication 35, **caractérisée par le fait qu'**elle possède un pH allant de 4 à 11.

40. Procédé de teinture des fibres kératiniques et en particulier des fibres kératiniques humaines telles que les cheveux, **caractérisé par le fait qu'**il consiste à appliquer sur les fibres au moins une composition A contenant, dans un milieu approprié pour la teinture, au moins un colorant d'oxydation, la couleur étant révélée à pH alcalin, neutre ou acide à l'aide d'une composition B contenant au moins un agent oxydant, qui est mélangée juste au moment de l'emploi à la composition A ou qui est appliquée séquentiellement sans rinçage intermédiaire, au moins un poly(vinyllactame) cationique tel que défini à l'une quelconque des revendications 1 à 14 étant présent dans la composition A ou dans la composition B ou dans chacune des compositions A et B et au moins un acide en C₁₀-C₁₄ tel que défini à l'une des revendications 15 à 17 et suivantes étant présent dans la composition A ou dans la composition B ou dans chacune des compositions A et B.

41. Procédé selon la revendication 40, **caractérisé par le fait qu'**il consiste à appliquer sur les fibres kératiniques sèches ou humides, la composition prête à l'emploi, réalisée extemporanément au moment de l'emploi à partir des compositions (A) et (B), à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

42. Dispositif à 2 compartiments ou « Kit » pour la teinture des fibres kératiniques et en particulier des fibres kératiniques humaines telles que les cheveux, **caractérisé par le fait qu'**un compartiment renferme une composition A1 contenant, dans un milieu approprié pour la teinture, au moins un colorant d'oxydation, et qu'un autre compartiment renferme une composition B1 contenant, dans un milieu approprié pour la teinture, un agent oxydant, au moins un poly(vinyllactame) cationique tel que défini à l'une quelconque des revendications 1 à 14 étant présent dans la composition A1 ou la composition B1, ou dans chacune des compositions A1 et B1 et l'acide gras en C₁₀-C₁₄ tel que défini à l'une quelconque des revendications 15 à 17 étant présent dans la composition A1 ou la composition B1, ou dans chacune des compositions A1 et B1.

43. Dispositif à 3 compartiments pour la teinture des fibres kératiniques et en particulier des fibres kératiniques humaines telles que les cheveux, **caractérisé par le fait qu'**un premier compartiment renferme une composition A2 contenant, dans un milieu approprié pour la teinture, soit au moins un colorant d'oxydation, un deuxième compartiment renferme une composition B2 contenant, dans un milieu approprié pour la teinture au moins un agent oxydant, et un troisième compartiment renferme une composition C contenant, dans un milieu approprié pour la teinture, au moins un poly(vinyllactame) cationique tel que défini à l'une quelconque des revendications 1 à 14, la composition A2 et/ou la composition B2 pouvant également contenir un poly(vinyllactame) cationique tel que défini aux revendications 1 à 14, la composition A2 et/ou la composition B2 et/ou la composition C pouvant également contenir un acide gras en C₁₀-C₁₄ tel que défini à l'une quelconque des revendications 15 à 17.

## Patentansprüche

1. Zusammensetzung zum oxidativen Färben von Keratinfasern und insbesondere menschlichen Keratinfasern, ganz besonders zum Färben der Haare, die in einem zum Färben geeigneten Medium mindestens einen Oxidationsfarbstoff, mindestens eine C₁₀₋₁₄-Fettsäure und mindestens ein kationisches Polyvinyllactampolymer enthält, das umfasst:
- a) mindestens ein Monomer vom Vinyllactamtyp oder Alkylvinyllactamtyp;
- b) mindestens ein Monomer der folgenden Strukturen (Ia) oder (Ib): 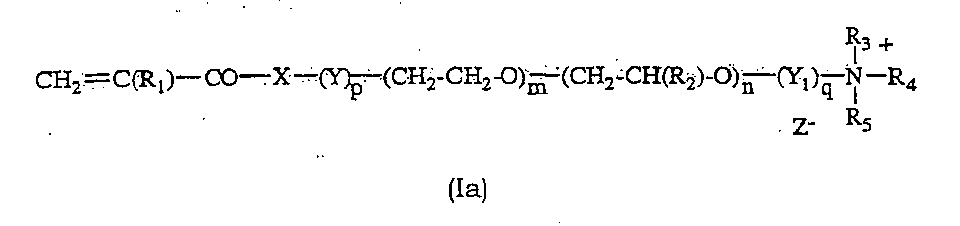 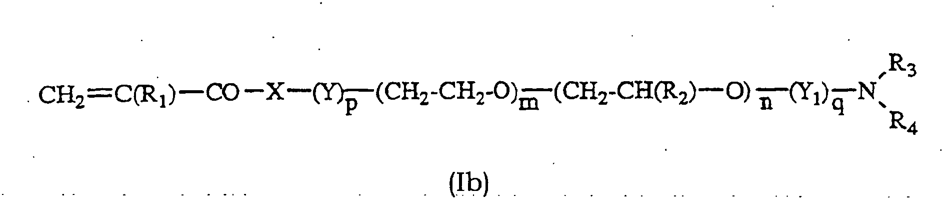
worin bedeuten:
X ein Sauerstoffatom oder eine Gruppe NR₆,
R₁ und R₆ unabhängig voneinander ein Wasserstoffatom oder eine geradkettige oder verzweigte C₁₋₅-Alkylgruppe,
R₂ eine geradkettige oder verzweigte C₁₋₄-Alkylgruppe,
R₃, R₄ und R₅ unabhängig voneinander ein Wasserstoffatom, eine geradkettige oder verzweigte C₁₋₃₀-Alkylgruppe oder eine Gruppe der Formel (II): 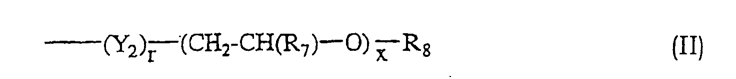
Y, Y₁ und Y₂ unabhängig voneinander eine geradkettige oder verzweigte C₂₋₁₆-Alkylengruppe,
R₇ ein Wasserstoffatom, eine geradkettige oder verzweigte C₁₋₄-Alkylgruppe oder eine geradkettige oder verzweigte C₁₋₄-Hydroxyalkylgruppe,
R₈ ein Wasserstoffatom oder eine geradkettige oder verzweigte C₁₋₃₀-Alkylgruppe,
p, q und r unabhängig voneinander entweder 0 oder 1,
m und n unabhängig eine ganze Zahl
von 0 bis 100,
x eine ganze Zahl von 1 bis 100,
Z ein Anion einer organischen oder anorganischen Säure, mit der Maßgabe, dass:
- mindestens ein Substituent R₃, R₄, R₅ oder R₈ eine geradkettige oder verzweigte C₉₋₃₀-Alkylkette bedeutet,
- q 1 ist, wenn m oder n von 0 verschieden ist,
- p oder q 0 bedeutet, wenn m oder n 0 ist.

2. Zusammensetzung nach Anspruch 1, **dadurch gekennzeichnet, dass** das Vinyllactammonomer oder_Alkylvinyllactammonomer eine Verbindung der Struktur III ist: 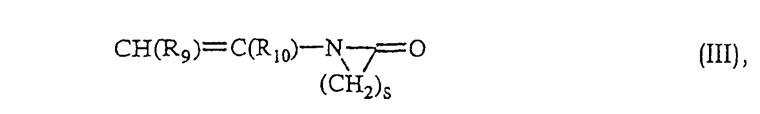 worin bedeuten:
s eine ganze Zahl von 3 bis 6,
R₉ ein Wasserstoffatom oder eine C₁₋₅-Alkylgruppe,
R₁₀ ein Wasserstoffatom oder eine C₁₋₅-Alkylgruppe,
mit der Maßgabe, dass mindestens eine der Gruppen R₉ und R₁₀ ein Wasserstoffatom bedeutet.

3. Zusammensetzung nach Anspruch 2, **dadurch gekennzeichnet, dass** das Monomer der Formel (III) das Vinylpyrrolidon ist.

4. Zusammensetzung nach einem der vorhergehenden Ansprüche, **dadurch gekennzeichnet, dass** in den Formeln (Ia) oder (Ib) die Gruppen R₃, R₄ und R₅ unabhängig voneinander ein Wasserstoffatom oder eine geradkettige oder verzweigte C₁₋₃₀-Alkylgruppe bedeuten.

5. Zusammensetzung nach einem der vorhergehenden Ansprüche, **dadurch gekennzeichnet, dass** das Monomer b) ein Monomer der Formel (Ia) ist.

6. Zusammensetzung nach Anspruch 5, **dadurch gekennzeichnet, dass** m und n in der Formel (Ia) Null bedeuten.

7. Zusammensetzung nach einem der vorhergehenden Ansprüche, **dadurch gekennzeichnet, dass** das Gegenion Z⁻ der Monomere der Formel (Ia) unter den Halogeniden, Phosphaten, Methosulfat oder Tosylat ausgewählt ist.

8. Zusammensetzung nach einem der Ansprüche 1 bis 7, **dadurch gekennzeichnet, dass** das oder die kationische(n) Poly(vinyllactam)polymer(e) ein oder mehrere zusätzliche, kationische oder nichtionische Monomere enthalten.

9. Zusammensetzung nach Anspruch 8, **dadurch gekennzeichnet, dass** das kationische Poly(vinyllactam) ein Terpolymer ist, das enthält:
a)- ein Monomer der Formel (III),
b) - ein Monomer der Formel (Ia) mit p = 1, q = 0, worin R₃ und R₄ unabhängig voneinander ein Wasserstoffatom oder eine C₁₋₅-Alkylgruppe bedeuten und R₅ eine C₉₋₂₄-Alkylgruppe ist und
c) - ein Monomer der Formel (Ib), worin R₃ und R₄ unabhängig voneinander ein Wasserstoffatom oder eine C₁₋₅-Alkylgruppe bedeuten.

10. Zusammensetzung nach Anspruch 9, **dadurch gekennzeichnet, dass** das Terpolymer 40 bis 95 Gew.-% Monomer (a), 0,25 bis 50 Gew.-% Monomer (b) und 0,1 bis 55 Gew.-% Monomer (c) enthält.

11. Zusammensetzung nach einem der vorhergehenden Ansprüche, **dadurch gekennzeichnet, dass** die kationischen Poly(vinyllactame) unter den Vinylpyrrolidon/Dimethylaminopropylmethacrylamid/Dodecyldimethylmethacrylamidopropylammoniumtosylat-Terpolymeren, Vinylpyrrolidon/ Dimethylaminopropylmethacrylamid/Cocoyldimethylmethacrylamidopropylammoniumtosylat-Terpolymeren, Vinylpyrrolidon/ Dimethylaminopropylmethacrylamid/Lauryldimethylmethacrylamidopropylammoniumtosylat-Terpolymeren oder Vinylpyrrolidon/Dimethylaminopropylmethacrylamid/Lauryldimethylmethacrylamidopropylammoniumchlorid-Terpolymeren ausgewählt sind.

12. Zusammensetzung nach einem der vorhergehenden Ansprüche, **dadurch gekennzeichnet, dass** die Molmasse der kationischen Poly(vinyllactame) im Bereich von 500 bis 20 000 000, vorzugsweise 200 000 bis 2 000 000 und noch bevorzugter 400 000 bis 800 000 liegt.

13. Zusammensetzung nach einem der vorhergehenden Ansprüche, **dadurch gekennzeichnet, dass** das oder die kationische(n) Poly(vinyllactam(e)) in einer Menge von 0,01 bis 10 Gew.-% des Gesamtgewichts der Zusammensetzung verwendet werden.

14. Zusammensetzung nach Anspruch 13, **dadurch gekennzeichnet, dass** das oder die kationische(n) Poly(vinyllactam(e)) in einer Menge von 0,1 bis 5 Gew.-% des Gesamtgewichts der Zusammensetzung verwendet werden.

15. Zusammensetzung nach einem der Ansprüche 1 bis 14, **dadurch gekennzeichnet, dass** die C₁₀₋₁₄-Fettsäure unter Caprinsäure, Laurinsäure und Myristinsäure und deren Gemischen ausgewählt ist.

16. Zusammensetzung nach Anspruch 15, **dadurch gekennzeichnet, dass** es sich bei der C₁₀₋₁₄-Fettsäure um die Laurinsäure handelt.

17. Zusammensetzung nach einem der vorhergehenden Ansprüche, **dadurch gekennzeichnet, dass** die C₁₀₋₁₄-Fettsäure(n) in einer Menge von 0,1 bis 40 Gew.-%, vorzugsweise 2 bis 25 Gew.-% und noch bevorzugter 5 bis 20 Gew.%, bezogen auf das Gesamtgewicht der Zusammensetzung, verwendet werden.

18. Zusammensetzung nach einem der vorhergehenden Ansprüche, **dadurch gekennzeichnet, dass** der Oxidationsfarbstoff unter den Oxidationsbasen und/oder den Kupplern ausgewählt ist.

19. Zusammensetzung nach Anspruch 18, **dadurch gekennzeichnet, dass** sie mindestens eine Oxidationsbase enthält.

20. Zusammensetzung nach den Ansprüchen 18 oder 19, **dadurch gekennzeichnet, dass** die Oxidationsbasen unter den p-Phenylendiaminen, Doppelbasen, o- oder p-Aminophenolen und den heterocyclischen Basen sowie den Additionssalzen dieser Verbindungen mit einer Säure ausgewählt sind.

21. Zusammensetzung nach einem der Ansprüche 18 bis 20, **dadurch gekennzeichnet, dass** die Oxidationsbasen jeweils in Konzentrationen von 0,0005 bis 20 Gew.-%, bezogen auf das Gesamtgewicht der Zusammensetzung, enthalten sind.

22. Zusammensetzung nach Anspruch 18, **dadurch gekennzeichnet, dass** die Kuppler unter den m-Phenylendiaminen, m-Aminophenolen, m-Dihydroxybenzolen, heterocyclischen Kupplern und den Additionssalzen dieser Verbindungen mit einer Säure ausgewählt sind.

23. Zusammensetzung nach einem der Ansprüche 18 oder 22, **dadurch gekennzeichnet, dass** die Kuppler jeweils in Konzentrationen von 0,0001 bis 20 Gew.-%, bezogen auf das Gesamtgewicht der Zusammensetzung, enthalten sind.

24. Zusammensetzung nach einem der Ansprüche 20 oder 22, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

25. Zusammensetzung nach einem der vorhergehenden Ansprüche, **dadurch gekennzeichnet, dass** sie ferner Direktfarbstoffe enthält.

26. Zusammensetzung nach einem der vorhergehenden Ansprüche, **dadurch gekennzeichnet, dass** sie ferner mindestens ein amphoteres Polymer oder ein kationisches Polymer, das von dem oder den kationischen Poly(vinyllactam(en)) nach einem der Ansprüche 1 bis 12 verschieden ist, enthält.

27. Zusammensetzung nach Anspruch 26, **dadurch gekennzeichnet, dass** das kationische Polymer ein Poly(quartäres Ammonium) ist, das aus wiederkehrenden Einheiten der folgenden Formel (W) besteht:

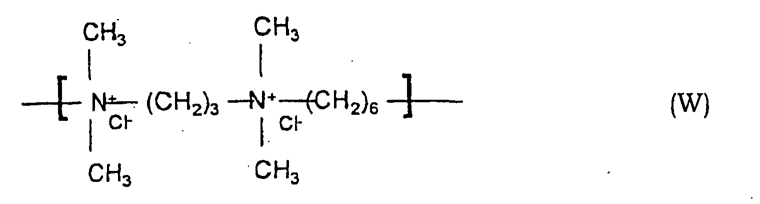

28. Zusammensetzung nach Anspruch 26, **dadurch gekennzeichnet, dass** das kationische Polymer ein Poly(quartäres Ammonium) ist, das aus wiederkehrenden Einheiten der folgenden Formel (U) besteht:

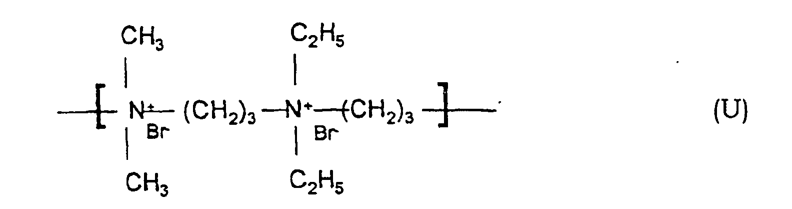

29. Zusammensetzung nach Anspruch 26, **dadurch gekennzeichnet, dass** das amphotere Polymer ein Copolymer ist, das als Monomer zumindest Acrylsäure und ein Dimethyldiallylammoniumsalz enthält.

30. Zusammensetzung nach einem der Ansprüche 26 bis 29, **dadurch gekennzeichnet, dass** das oder die kationische(n) oder amphotere(n) Polymer(e) 0,01 bis 10 Gew.-%, vorzugsweise 0,05 bis 5 Gew.-% und noch bevorzugter 0,1 bis 3 Gew.% des Gesamtgewichts der Zusammensetzung ausmachen.

31. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren grenzflächenaktiven Stoffen ausgewählt ist.

32. Zusammensetzung nach Anspruch 31, **dadurch gekennzeichnet, dass** die grenzflächenaktiven Stoffe 0,01 bis 40 Gew.-% und vorzugsweise 0,5 bis 30 Gew.-% des Gesamtgewichts der Zusammensetzung ausmachen.

33. Zusammensetzung nach einem der vorhergehenden Ansprüche, **dadurch gekennzeichnet, dass** sie mindestens ein zusätzliches Verdickungsmittel enthält.

34. Zusammensetzung nach einem der vorhergehenden Ansprüche, **dadurch gekennzeichnet, dass** sie ferner mindestens ein Reduktionsmittel in Mengenanteilen von 0,05 bis 3 Gew.%, bezogen auf das Gesamtgewicht der Zusammensetzung, enthält.

35. Zusammensetzung nach einem der vorhergehenden Ansprüche, **dadurch gekennzeichnet, dass** sie ferner ein Oxidationsmittel enthält und **dadurch**, dass sie gebrauchsfertig ist.

36. Zusammensetzung nach Anspruch 35, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen gegebenenfalls zusammen mit ihrem jeweiligen Donor oder Cofaktor ausgewählt ist.

37. Zusammensetzung nach Anspruch 36, **dadurch gekennzeichnet, dass** das Oxidationsmittel Wasserstoffperoxid ist.

38. Zusammensetzung nach Anspruch 37, **dadurch gekennzeichnet, dass** das Oxidationsmittel eine Wasserstoffperoxidlösung ist, deren Titer im Bereich von 1 bis 40 Volumina liegt.

39. Zusammensetzung nach Anspruch 35, **dadurch gekennzeichnet, dass** sie einen pH-Wert von 4 bis 11 aufweist.

40. Verfahren zum Färben von Keratinfasern und insbesondere menschlichen Keratinfasern, wie Haaren, **dadurch gekennzeichnet, dass** es darin besteht, auf die Fasern mindestens eine Zusammensetzung A aufzutragen, die in einem zum Färben geeigneten Medium mindestens einen Oxidationsfarbstoff enthält, wobei die Farbe bei einem alkalischen, neutralen oder sauren pH-Wert mit einer Zusammensetzung B entwickelt wird, die ein Oxidationsmittel enthält und die bei der Anwendung mit der Zusammensetzung A vermischt wird oder die ohne zwischenzeitliches Spülen anschließend aufgetragen wird, wobei mindestens ein kationisches Poly(vinyllactam) nach einem der Ansprüche 1 bis 14 in der Zusammensetzung A oder in der Zusammensetzung B oder in beiden Zusammensetzungen A und B enthalten ist und mindestens eine C₁₀₋₁₄-Säure nach einem der Ansprüche 15 bis 17 in der Zusammensetzung A oder in der Zusammensetzung B oder in beiden Zusammensetzungen A und B enthalten ist.

41. Verfahren nach Anspruche 40, **dadurch gekennzeichnet, dass** es darin besteht, die bedarfsgemäß bei der Anwendung aus den Zusammensetzungen A und B hergestellte gebrauchsfertige Zusammensetzung auf die trockenen oder feuchten Keratinfasern aufzutragen, während einer Einwirkzeit von etwa 1 bis 60 Minuten und vorzugsweise 10 bis 45 Minuten einwirken zu lassen, die Fasern zu spülen, dann gegebenenfalls mit Haarwaschmittel zu waschen, nochmals zu spülen und zu trocknen.

42. Vorrichtung mit zwei Abteilungen oder "Kit" zum Färben von Keratinfasern und insbesondere menschlichen Keratinfasern, wie Haaren, **dadurch gekennzeichnet, dass** eine Abteilung eine Zusammensetzung A1, die in einem zum Färben geeigneten Medium mindestens einen Oxidationsfarbstoff enthält, und eine andere Abteilung eine Zusammensetzung B1 enthält, die in einem zum Färben geeigneten Medium ein Oxidationsmittel enthält, wobei mindestens ein kationisches Poly(vinylläctam) nach einem der Ansprüche 1 bis 14 in der Zusammensetzung A1 oder in der Zusammensetzung B1 oder in beiden Zusammensetzungen A1 und B1 und eine C₁₀₋₁₄-Säure nach einem der Ansprüche 15 bis 17 in der Zusammensetzung A1 oder in der Zusammensetzung B1 oder in beiden Zusammensetzungen A1 und B1 enthalten ist.

43. Vorrichtung mit drei Abteilungen zum Färben von Keratinfasern und insbesondere menschlichen Keratinfasern, wie Haaren, **dadurch gekennzeichnet, dass** eine Abteilung eine Zusammensetzung A2 enthält, die in einem zum Färben geeigneten Medium mindestens einen Oxidationsfarbstoff enthält, eine zweite Abteilung eine Zusammensetzung B2 enthält, die in einem zum Färben geeigneten Medium mindestens ein Oxidationsmittel enthält, und eine dritte Abteilung eine Zusammensetzung C enthält, die in einem zum Färben geeigneten Medium mindestens ein kationisches Poly(vinyllactam) nach einem der Ansprüche 1 bis 14 enthält, wobei die Zusammensetzung A2 und/oder die Zusammensetzung B2 ebenfalls ein kationisches Poly(vinyllactam) nach einem der Ansprüche 1 bis 14 enthalten können und wobei die Zusammensetzung A2 und/oder die Zusammensetzung B2 und/oder die Zusammensetzung C eine C₁₀₋₁₄-Fettsäure nach einem der Ansprüche 15 bis 17 enthalten können.

## Claims

1. Composition for the oxidation dyeing of keratin fibres, in particular human keratin fibres, and more particularly hair, comprising, in an appropriate dyeing medium, at least one oxidation dye, at least one C₁₀-C₁₄ fatty acid and at least one cationic poly(vinyllactam) polymer comprising:
-a) at least one monomer of the vinyllactam or alkylvinyllactam type;
-b) at least one monomer having the following structures (Ia) or (Ib): 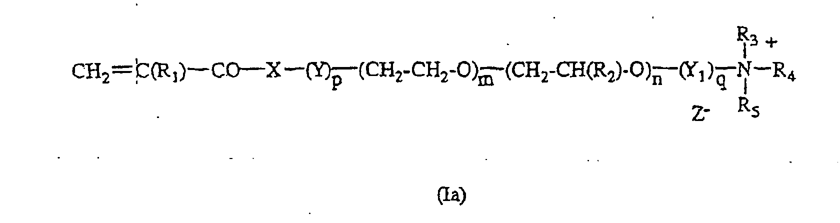 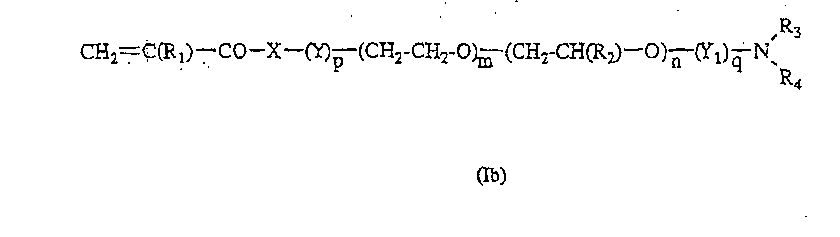 in which:
X denotes an oxygen atom or a radical NR₆,
R₁ and R₆ denote, independently of each other, a hydrogen atom or a linear or branched C₁-C₅ alkyl radical,
R₂ denotes a linear or branched C₁₋₄ alkyl radical, R₃, R₄ and R₅ denote, independently of each other, a hydrogen atom, a linear or branched C₁-C₃₀ alkyl radical or a radical of formula (II): 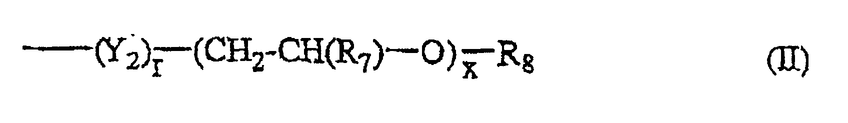 Y, Y₁ and Y₂ denote, independently of each other, a linear or branched C₂-C₁₆ alkylene radical,
R₇ denotes a hydrogen atom, or a linear or branched C₁-C₄ alkyl radical or a linear or branched C₁-C₄ hydroxyalkyl radical,
R₈ denotes a hydrogen atom or a linear or branched C₁-C₃₀ alkyl radical,
p, q and r denote, independently of each other, either the value zero, or the value 1,
m and n denote, independently of each other, an integer ranging from 0 to 100,
x denotes an integer ranging from 1 to 100,
Z denotes an organic or inorganic acid anion, provided that:
- at least one of the substituents R₃, R₄, R₅ or R₈ denotes a linear or branched C₉-C₃₀ alkyl radical,
- if m or n is different from zero, then q is equal to 1,
- if m or n are equal to zero, then p or q is equal to 0.

2. Composition according to Claim 1, **characterized in that** the vinyllactam or alkylvinyllactam monomer is a compound having the structure (III): 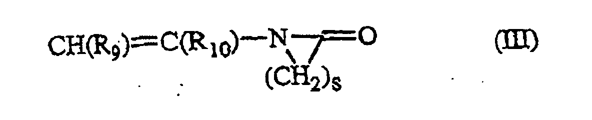 in which:
s denotes an integer ranging from 3 to 6,
R₉ denotes a hydrogen atom or a C₁-C₅ alkyl radical,
R₁₀ denotes a hydrogen atom or a C₁-C₅ alkyl radical,
provided that at least one of the radicals R₉ and R₁₀ denotes a hydrogen atom.

3. Composition according to Claim 2, **characterized in that** the monomer of formula (III) is vinylpyrrolidone.

4. Composition according to any one of the preceding claims, **characterized in that** in formulae (Ia) or (Ib), the radicals R₃, R₄ and R₅ denote, independently of each other, a hydrogen atom or a linear or branched C₁-C₃₀ alkyl radical.

5. Composition according to any one of the preceding claims, **characterized in that** the monomer b) is a monomer of formula (Ia).

6. Composition according to Claim 5, **characterized in that** in formula (Ia), m and n are equal to zero.

7. Composition according to any one of the preceding claims, **characterized in that** the counterion Z⁻ of the monomers of formula (Ia) is chosen from the halide ions, the phosphate ions, the methosulphate ion and the tosylate ion.

8. Composition according to any one of Claims 1 to 7, **characterized in that** the cationic poly(vinyllactam) polymer(s) contain one or more additional cationic or nonionic monomers.

9. Composition according to Claim 8, **characterized in that** the cationic poly(vinyllactam) is a terpolymer comprising:
a) one monomer of formula (III),
b) one monomer of formula (Ia) in which p = 1, q = 0, R₃ and R₄ denote, independently of each other, a hydrogen atom or a C₁-C₅ alkyl radical and R₅ denotes a C₉-C₂₄ alkyl radical, and
c) one monomer of formula (Ib) in which R₃ and R₄ denote, independently of each other, a hydrogen atom or a C₁-C₅ alkyl radical.

10. Composition according to Claim 9, **characterized in that** the terpolymer comprises, by weight, 40 to 95% of monomer (a), 0.25 to 50% of monomer (b) and 0.1 to 55% of monomer (c).

11. Composition according to any one of the preceding claims, **characterized in that** the cationic poly(vinyllactams) are chosen from the terpolymers vinylpyrrolidone/dimethylaminopropylmethacrylamide /dodecyldimethylmethacrylamidopropylammonium tosylate, the terpolymers vinylpyrrolidone/dimethylaminopropylmethacrylamide/cocoyldimethylmethacrylamidopropylammonium tosylate, the terpolymers vinylpyrrolidone/dimethylaminopropylmethacrylamide/lauryldimethylmethacrylamidopropylammonium tosylate or chloride.

12. Composition according to any one of the preceding claims, **characterized in that** the weight-average molecular mass of the cationic poly(vinyllactams) is between 500 and 20 000 000, preferably between 200 000 and 2 000 000 and more preferably between 400 000 and 800 000.

13. Composition according to any one of the preceding claims, **characterized in that** the cationic poly(vinyllactam) or poly(vinyllactams) are used in a quantity varying from 0.01 to 10% by weight of the total weight of the composition.

14. Composition according to Claim 13, **characterized in that** the cationic poly(vinyllactam) or poly-(vinyllactams) are used in a quantity varying from 0.1 to 5% by weight of the total weight of the composition.

15. Composition according to any one of Claims 1 to 14, **characterized in that** the C₁₀-C₁₄ fatty acid is chosen from the group formed by capric, lauric and myristic acids, and mixtures thereof.

16. Composition according to Claim 15, **characterized in that** the C₁₀-C₁₄ fatty acid is lauric acid.

17. Composition according to any one of the preceding claims, **characterized in that** the C₁₀-C₁₄ fatty acid(s) are used in a quantity ranging from 0.1 to 40%, preferably from 2 to 25% and still more preferably from 5 to 20% by weight relative to the total weight of the composition.

18. Composition according to any one of the preceding claims, **characterized in that** the oxidation dye is chosen from oxidation bases and/or couplers.

19. Composition according to Claim 18, **characterized in that** it contains at least one oxidation base.

20. Composition according to Claim 18 or 19, **charac** **terized in that** the oxidation bases are chosen from para-phenylenediamines, double bases, ortho- or para-aminophenols, and heterocyclic bases, and the addition salts of these compounds with an acid.

21. Composition according to any one of Claims 18 to 20, **characterized in that** the oxidation bases are each present in concentrations ranging from 0.0005 to 20% by weight relative to the total weight of the composition.

22. Composition according to Claim 18, **characterized in that** the couplers are chosen from metaphenylenediamines, meta-aminophenols, metadiphenols, heterocyclic couplers, and the addition salts of these compounds with an acid.

23. Composition according to either of Claims 18 and 22, **characterized in that** the couplers are each present in concentrations ranging from 0.0001 to 20% by weight relative to the total weight of the composition.

24. Composition according to either of Claims 20 and 22, **characterized in that** the addition salts with an acid of the oxidation dyes are chosen from hydrochlorides, hydrobromides, sulphates, tartrates, lactates and acetates.

25. Composition according to any one of the preceding claims, **characterized in that** it contains, in addition, direct dyes.

26. Composition according to any one of the preceding claims, **characterized in that** it contains in addition at least one amphoteric polymer or one cationic polymer different from the cationic poly(vinyllactam) or poly(vinyllactams) as defined in any one of Claims 1 to 12.

27. Composition according to Claim 26, **characterized in that** the cationic polymer is a quaternary polyammonium consisting of recurring units corresponding to the following formula (W):

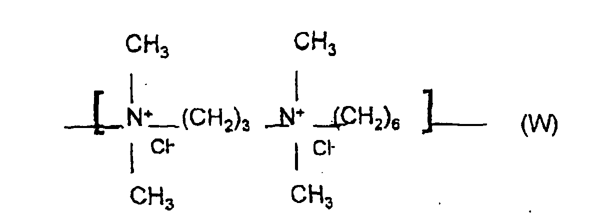

28. Composition according to Claim 26, **characterized in that** the cationic polymer is a quaternary polyammonium consisting of recurring units corresponding to the following formula (U):

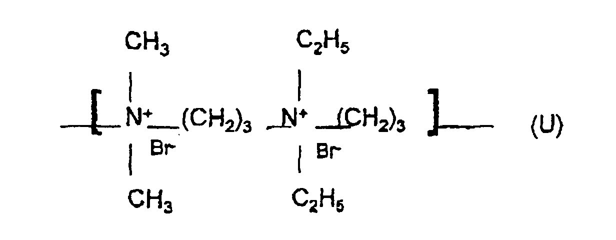

29. Composition according to Claim 26, **characterized in that** the amphoteric polymer is a copolymer comprising at least as monomers acrylic acid and a salt of dimethyldiallylammonium.

30. Composition according to any one of Claims 26 to 29, **characterized in that** the cationic or amphoteric polymer(s) represent from 0.01% to 10%, preferably from 0.05% to 5%, and even more preferably from 0.1% to 3% by weight, of the total weight of the composition.

31. Composition according to any one of the preceding claims, **characterized in that** it contains at least one surfactant chosen from anionic, cationic, nonionic or amphoteric surfactants.

32. Composition according to Claim 31, **characterized in that** the surfactants represent 0.01 to 40% and preferably from 0.5 to 30% by weight, of the total weight of the composition.

33. Composition according to any one of the preceding claims, **characterized in that** it contains at least one additional thickening agent.

34. Composition according to any one of the preceding claims, **characterized in that** it contains in addition at least one reducing agent, in quantities ranging from 0.05 to 3% by weight relative to the total weight of the composition.

35. Composition according to any one of the preceding claims, **characterized in that** it contains in addition an oxidizing agent and **in that** it is ready for use.

36. Composition according to Claim 35, **characterized in that** the oxidizing agent is chosen from hydrogen peroxide, urea peroxide, alkali metal bromates or ferricyanides, persalts, oxidation-reduction enzymes with optionally their respective donor or cofactor.

37. Composition according to Claim 36, **characterized in that** the oxidizing agent is hydrogen peroxide.

38. Composition according to Claim 37, **characterized in that** the oxidizing agent is a hydrogen peroxide solution whose titre varies from 1 to 40 volumes.

39. Composition according to Claim 35, **characterized in that** it has a pH ranging from 4 to 11.

40. Method for dyeing keratin fibres, and in particular human keratin fibres such as hair, **characterized in that** it consists in applying to the fibres at least one composition A containing, in an appropriate dyeing medium, at least one oxidation dye, the colour being developed at alkaline, neutral or acidic pH with the aid of a composition B containing at least one oxidizing agent, which is mixed just at the time of use with the composition A or which is applied sequentially without intermediate rinsing, at least one cationic poly(vinyllactam) as defined in any one of Claims 1 to 14 being present in the composition A or in the composition B or in each of the compositions A and B and at least one C₁₀-C₁₄ acid as defined in one of Claims 15 to 17 and subsequent claims being present in the composition A or in the composition B or in each of the compositions A and B.

41. Method according to Claim 40, **characterized in that** it consists in applying the ready-to-use composition, freshly prepared at the time of use from the compositions (A) and (B) described above, to the dry or wet keratin fibres, and in allowing it to act for an exposure time varying from 1 to 60 minutes approximately, and preferably from 10 to 45 minutes, in rinsing the fibres, and then optionally in washing them with shampoo, and then in rinsing them again, and in drying them.

42. Two-compartment device or "kit" for dyeing keratin fibres and in particular human keratin fibres such as hair, **characterized in that** one compartment contains a composition A1 containing, in an appropriate dyeing medium, at least one oxidation dye, and **in that** another compartment contains a composition B1 containing, in an appropriate dyeing medium, an oxidizing agent, at least one cationic poly(vinyllactam) as defined in any one of Claims 1 to 14 being present in the composition A1 or the composition B1, or in each of the compositions A1 and B1 and the C₁₀-C₁₄ fatty acid as defined in any one of Claims 15 to 17 being present in the composition A1 or the composition B1, or in each of the compostiions A1 and B1.

43. Three-compartment device for dyeing keratin fibres and in particular human keratin fibres such as hair, **characterized in that** a first compartment contains a composition A2 containing, in an appropriate dyeing medium, either at least one oxidation dye, a second compartment contains a composition B2 containing, in an appropriate dyeing medium, at least one oxidizing agent, and a third compartment contains a composition C containing, in an appropriate dyeing medium, at least one cationic poly(vinyllactam) as defined in any one of Claims 1 to 14, it being also possible for the composition A2 and/or the composition B2 to contain a cationic poly(vinyllactam) as defined in Claims 1 to 14, it being also possible for the composition C to contain a C₁₀-C₁₄ fatty acid as defined in any one of Claims 15 to 17.
